(19)

Europäisches Patentamt

European Patent Office

Office européen des brevets

(11) **EP 4 079 353 B1**

(12) **EUROPEAN PATENT SPECIFICATION**

(45) Date of publication and mention
of the grant of the patent:
**19.03.2025   Bulletin 2025/12**

(21) Application number: **19956676.1**

(22) Date of filing: **16.12.2019**

(51) International Patent Classification (IPC):
**A61M 16/00** *(2006.01)*      **A61B 5/08** *(2006.01)*

(52) Cooperative Patent Classification (CPC):
(C-Sets available)
**A61M 16/0003; A61M 16/0051; G16H 20/40;
G16H 40/63;** A61B 5/08; A61B 5/4836;
A61M 16/0833; A61M 16/0891; A61M 16/104;
A61M 16/109; A61M 16/12; A61M 16/18;
A61M 16/204; A61M 16/205; A61M 16/208;   (Cont.)

(86) International application number:
**PCT/CN2019/125645**

(87) International publication number:
**WO 2021/119919 (24.06.2021 Gazette 2021/25)**

(54) **DEVICE, AND MEDICAL DEVICE APPARATUS FOR MEDICAL VENTILATION**

VORRICHTUNG ZUR ANZEIGE DER BEATMUNGSINFORMATIONEN FÜR EINE MEDIZINISCHE
BEATMUNGSVORRICHTUNG

APPAREIL D'AFFICHAGE D'INFORMATIONS DE VENTILATION POUR DISPOSITIF DE
VENTILATION MÉDICAL, ET DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.10.2022   Bulletin 2022/43**

(73) Proprietor: **Shenzhen Mindray Bio-Medical
Electronics Co., Ltd.
Shenzhen, Guangdong 518057 (CN)**

(72) Inventors:
• **PAN, Ruiling
  Shenzhen, Guangdong 518057 (CN)**

• **WANG, Huihua
  Shenzhen, Guangdong 518057 (CN)**
• **CHEN, Yu
  Shenzhen, Guangdong 518057 (CN)**

(74) Representative: **KIPA AB
Drottninggatan 11
252 21 Helsingborg (SE)**

(56) References cited:
**WO-A1-2011/075601      WO-A1-2014/124684
WO-A2-2007/144767      WO-A2-2008/039726
CN-A- 102 458 544      CN-A- 106 462 660
CN-A- 107 660 132      CN-A- 110 520 182
US-A1- 2007 199 566      US-A1- 2008 072 896
US-A1- 2011 154 241**

(52) Cooperative Patent Classification (CPC): (Cont.)
A61M 16/22; A61M 2016/0027; A61M 2016/0039;
A61M 2016/0042; A61M 2202/0208;
A61M 2202/0283; A61M 2205/07; A61M 2205/18;
A61M 2205/3334; A61M 2205/3344;
A61M 2205/3584; A61M 2205/3592;
A61M 2205/502; A61M 2205/505; A61M 2205/52;
A61M 2205/584; A61M 2205/8206;
A61M 2205/8262; A61M 2210/1014;
A61M 2210/1021; A61M 2210/105;
A61M 2210/1053; A61M 2230/04; A61M 2230/205;
A61M 2230/30; A61M 2230/42; A61M 2230/46;
A61M 2230/50

C-Sets
A61M 2202/0208, A61M 2202/0007;
A61M 2202/0283, A61M 2202/0007;

A61M 2230/04, A61M 2230/005;
A61M 2230/205, A61M 2230/005;
A61M 2230/30, A61M 2230/005;
A61M 2230/42, A61M 2230/005;
A61M 2230/46, A61M 2230/005;
A61M 2230/50, A61M 2230/005

**Description**

TECHNICAL FIELD

The disclosure relates to a medical device comprising a ventilation information display apparatus as defined by the appended claims.

BACKGROUND

Human respiration refers to periodic rhythmic inhalation and exhalation of gas, absorption of oxygen and discharge of carbon dioxide, which are for gas exchange. When some patients are unable to breathe spontaneously, mechanical ventilation can be configured to help them finish respiration. For example, if the patient does not breathe spontaneously, external devices, such as a ventilator can usually be configured to provide ventilatory support to the patient. It can be seen that mechanical ventilation is a ventilation method that uses a mechanical device to replace, control or change the spontaneous ventilation action of the patient.

With the development of mechanical ventilation technology, increased ventilation modes appear, such as assisted ventilation (AV) mode, controlled ventilation (CV) mode, assisted-control ventilation (A-CV) mode, intermittent mandatory ventilation (IMV) mode and mandatory minute ventilation (MMV) mode, and the likes.

Take the mandatory minute ventilation mode as an example. In the mandatory minute ventilation mode, the ventilation device, such as a ventilator, ventilates the patient according to a predetermined ventilation volume per minute. If the spontaneous ventilation volume of the patient is lower than a preset ventilation volume per minute, the insufficient part is provided by the ventilator. If the spontaneous ventilation volume of the patient is greater than or equal to the preset ventilation volume per minute, the ventilator is no longer supplying gas. Clinically, the mandatory minute ventilation mode is mainly to guarantee the smooth transition from controlled ventilation to spontaneous respiration and to avoid the occurrence of insufficient ventilation, when the ventilator is removed from the patient.

At present, there is still improvement space for some mechanical ventilation modes, such as the mandatory minute ventilation mode. WO2008/039726 describes a ventilator system comprising a programmable ventilator configured to ventilate a patient based on settings for a plurality of ventilation parameters.

SUMMARY

This disclosure mainly provides a ventilation information display method for a medical ventilation device, a ventilation information display apparatus, and a medical device.

According to a first aspect, not part of the invention, an embodiment provides a ventilation information display method for a medical ventilation device which is configured to provide respiratory support for a patient, wherein the ventilation information display method includes

> acquiring at least one respiratory parameter of the patient who is connected to the medical ventilation device;
> acquiring a target of a ventilation control parameter;
> acquiring a ventilation treatment strategy;
> calculating a target of at least one associated control parameter according to the target of the ventilation control parameter and the ventilation treatment strategy;
> obtaining, according to the acquired respiratory parameter, a ventilation parameter which characterizes a ventilation state of the patient; and
> generating and outputting a visual graph according to the ventilation parameter of the patient, the target of the ventilation control parameter and the target of the at least one associated control parameter, wherein the ventilation state of the patient, the target of the ventilation control parameter and the target of the at least one associated control parameter are displayed through the visual graph.

According to a second aspect, not part of the invention, an embodiment provides a ventilation information display apparatus, which including:

> a display;
> one or more sensors, which is/are configured to acquire at least one respiratory parameter of a patient; and
> a processor, which is configured to implement the above ventilation information display method according to any embodiment of this disclosure.

According to a third aspect, an embodiment provides a medical device, which includes the ventilation information display apparatus according to any embodiment of this disclosure.

According to a fourth aspect, not part of the invention, an embodiment provides a computer-readable storage medium which includes a program, wherein when the program is executed by a processor, the above ventilation information display method according to any embodiment of this disclosure is implemented.

BRIEF DESCRIPTION OF THE DRAWINGS

FIG. 1 is a structural diagram of a medical device according to an embodiment.

FIG. 2 is a structural diagram of a medical device according to another embodiment.

FIG. 3 is a structural diagram of a medical device according to a further embodiment.

FIG. 4 is a structural diagram of a medical device according to another further embodiment.

FIG. 5 (a), FIG. 5 (b) and FIG. 5 (c) are three examples in which the visual graphs are cyclic graphs, respectively.

FIG. 6 is an example which simultaneously displays visual graphs which are corresponding to ventilation volume per minute, tidal volume, and respiratory rate.

FIG. 7 (a), FIG. 7 (b) and FIG. 7 (c) are three examples in which the visual graphs are histograms, respectively.

FIG. 8 is an example which simultaneously displays visual graphs which are corresponding to ventilation volume per minute, tidal volume, and respiratory rate.

FIG. 9 is an example which simultaneously displays visual graphs which are corresponding to ventilation volume per minute, tidal volume, and respiratory rate.

FIG. 10 is an example which simultaneously displays visual graphs which are corresponding to ventilation volume per minute, tidal volume, and respiratory rate.

FIG. 11 is an example in which the visual graph is a two-dimensional curve.

FIG. 12 is an example which simultaneously displays visual graphs which are corresponding to ventilation volume per minute, tidal volume, and respiratory rate.

FIG. 13 is an example which simultaneously displays visual graphs which are corresponding to ventilation volume per minute, tidal volume, and respiratory rate.

FIG. 14 is an example in which the visual graph is an altimeter graph.

FIG. 15 is a flowchart of a ventilation information display method according to an embodiment.

DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are operable to distinguish different objects, rather than to describe a specific order. In addition, the terms "connection", and "linkage" mentioned in this application include direct and indirect connection (linkage), unless otherwise specified.

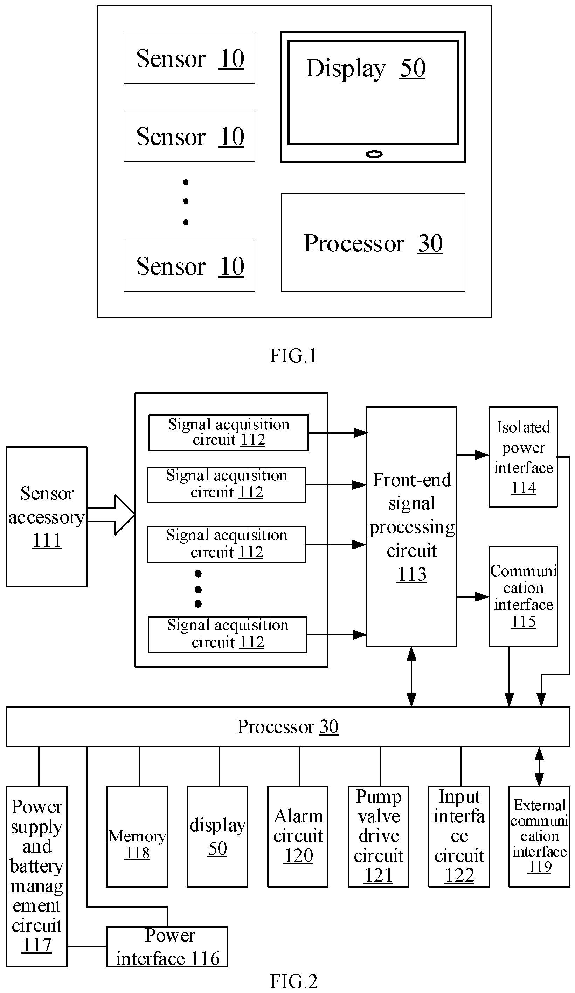

Some embodiments of the disclosure discloses a medical device, which may include a ventilation information display apparatus. Referring to FIG. 1, in some embodiments, the ventilation information display apparatus may include one or more sensors 10, a processor 30, and a display 50. The medical device in this disclosure may be a patient monitor or a patient monitoring module in some embodiments, and may be a medical ventilation device in some embodiments, such as a ventilator and an anesthetic machine, and the likes. When the medical device includes a ventilation information display apparatus, correspondingly, the medical device can be a patient monitor with a ventilation information display apparatus, a patient monitoring module with a ventilation information display apparatus, and a medical ventilation device with a ventilation information display apparatus. Or, to put it another way, the medical device can be a patient monitor with ventilation information display function, a patient monitoring module with ventilation information display function, and a medical ventilation device with ventilation information display function, which are described below.

Referring FIG.2, in some embodiments, the medical device has an independent housing, whose panel is provided with a sensor interface zone which is integrated therein multiple sensor interfaces for connecting with various external physiological parameter sensor accessories 111, or in some embodiment for connecting with the above sensor 10. The housing panel also includes one or more of a small IxD display zone, an input interface circuit 122 and an alarm circuit 120 (such as, a LED alarm zone) and the likes. The medical device includes an external communication and power interface 116 for communicating with a main unit of a medical device (such as a patient monitor, ventilator, anesthetic machine, etc.) and taking power from the main unit. The medical device also supports a build-out parameter module, can form a plug-in monitor main unit by means of inserting the parameter module, can be used as part of the monitor, or can be connected to the main unit via a cable, with the build-out parameter module being used as an external accessory of the monitor. The internal circuit of the medical device is disposed inside the housing. The internal circuit includes a signal acquisition circuit 112 corresponding to one or more physiological parameters, and a front-end signal processing circuit 113. The signal acquisition circuit 112 may be selected from an electrocardiogram circuit, a body temperature circuit, a blood oxygen saturation circuit, a non-invasive blood pressure circuit, an invasive blood pressure circuit, etc. These signal acquisition circuits 112 are electrically connected with corresponding sensor interfaces for electrically connecting to sensor accessories 111 corresponding to different physiological parameters. An output terminal of the signal acquisition circuit 112 is coupled to the front-end signal processing circuit 113 whose communication terminal is further coupled to the processor 30. The processor 30 is electrically connected with the external communication and power interface 116. Various general circuits known in the prior art can be configured to realize the signal acquisition circuit 112 and sensor accessories 111 corresponding to different physiological parameters. The front-end signal processing circuit 113 can be configured to complete the sampling and analog-to-digital conversion of output signals of the signal acquisition circuit 112, and output control signals to control the physiological parameter measurement process. The physiological parameters include but are not limited to parameters of electrocardiogram, body temperature, blood oxygen saturation, non-invasive blood pressure, and invasive blood pressure. The front-end signal processing circuit113 can be realized by a single-chip microcomputer or other semiconductor devices, such as mixed signal single-chip microcomputer of LPC2136 which is produced by PHLIPS Corp., or ADuC7021 which is produced by ADI Corp, or by ASIC or FPGA. The front-end signal processing circuit 113 can be powered by an isolated power supply. After a simple processing and packaging, the sampled data can be sent to the processor 30 through an isolated communication interface. For example, the front-end signal processing circuit 113 can be coupled to the processor 30 through an isolated power interface 114 and a communication interface 115. Supplying electrical power to the front-end signal processing circuit through the isolated power supply has a function of isolating the patient from the power supply device through isolating the DC/DC power supply via a transformer. In such a way, 1, the application part is floating through the isolation transformer, such that the leakage current passing through the patient is small enough, and 2, bad influences on boards and devices of intermediate circuits, such as main control board (guaranteed by creepage distance and electrical clearance), due to voltage or energy generated during a defibrillation or electric knife application, can be prevented. Of course, the front-end signal processing circuit 113 can be coupled to the processor 30 directly through cables. The processor 30 completes the calculation of physiological parameters and sends calculation results and waveforms of the physiological parameter to the main unit (such as, a main unit with a display, PC, a central station, etc.) through the external communication interface 119. The processor 30 can be directly connected with the external communication interface 119 through cables for communication and be directly connected with the external communication and power source interface 116 through cables for taking power. The medical device may also include a power supply and battery management circuit 117, which takes power from the main unit through the external communication and power interface 116 and supplies it to the processor 30 after processing, such as rectification and filtering. The power supply and battery management circuit 117 can also monitor, manage and protect the power obtained from the main unit through the external communication and power interface 116. The external communication interface 119 may be one or a combination of local area network interfaces composed of Ethernet, Token Ring, Token Bus, and optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication interface 119 may also be one or a combination of the wireless data transmission interface and the wired data transmission interface. The main unit may be any computer device such as a main unit of the monitor, a computer, and the likes, and a monitor can be formed by means of installing with matching software on the main unit. The main unit may also be a communication device, such as a mobile phone, and the medical device sends data to a mobile phone that supports Bluetooth communication via a Bluetooth interface to realize remote data transmission. After the processor 30 completes the calculation of physiological parameters, it can also determine whether the physiological parameters are abnormal. If yes, it can alarm through the alarm circuit 120. The memory 118 may store intermediate and final data of the monitor, as well as program instructions or code for execution by the processor 30 or the like. If the monitor has the function of blood pressure measurement, it may also include a pump valve drive circuit 121, which is configured to implement inflation or deflation under the control of the processor 30.

The above are some descriptions of medical device as a patient monitor.

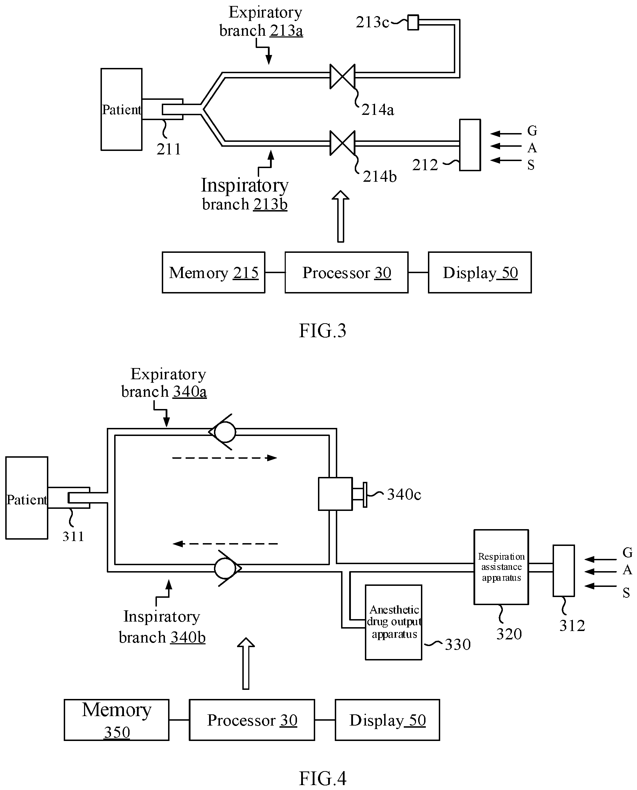

In some embodiments, the medical device can also be a ventilator, which is an artificial mechanical ventilation apparatus to assist or control the spontaneous respiration movement of the patient, so as to achieve the function of gas exchange in the lung, reduce the consumption of the human body, and facilitate the recovery of respiratory function. Referring to FIG. 3, in some embodiments, the medical device may also include a respiratory interface 211, a gas source interface 212, a respiratory line, and a respiration assist apparatus. The respiratory line selectively connects the gas source interface 212 with the respiratory system of the patient. In some embodiments, the respiratory line includes an inspiratory branch 213b and an expiratory branch 213a. The expiratory branch 213a is connected between the respiratory interface 211 and an exhaust port 213c, and is configured to export the exhaled gas of the patient to the exhaust port 213c. The exhaust port 213c can be connected to the external environment, and can also be in a dedicated gas recovery apparatus. The gas source interface 212 is configured to connect with the gas source (not shown), which is configured to provide gas. The gas can usually be oxygen, air and so on. In some embodiments, the gas source can be a compressed gas cylinder or a central gas supply source, which supplies gas to the ventilator through the gas source interface 212. The types of gas include oxygen $O_2$, air, etc. The gas source interface 212 can include conventional components, such as pressure gauge, pressure regulator, flowmeter, pressure-reduction valve and air- oxygen proportional control protection device, which are respectively configured to control the flow of various gases (such as oxygen and air). The inspiratory branch 213b is connected between the respiratory interface 211 and the gas source interface 212 to provide oxygen or air for the patient. For example, the gas which is inputted from the gas source interface 212, enters the inspiratory branch 213b, and then enters the lungs of the patient through the respiratory interface 211. The respiratory interface 211 is configured to connect the patient to the respiratory line. In addition to guiding the gas transmitted by the inspiratory branch 213b to the patient, the exhaled gas of the patient can also be guided to the exhaust port 213c through the expiratory branch 213a. Depending on the situation, the respiratory interface 211 may be a nasal cannula or a mask for wearing on the mouth and nose. The respiration assistance apparatus is connected with the gas source interface 212 and the respiratory line, and controls the gas provided by the external gas source to be transmitted to the patient through the respiratory line. In some embodiments, the respiration assistance apparatus may include an expiratory controller 214a and an inspiratory controller 214b. The expiratory controller 214a is arranged on the expiratory branch 213a to switch on or off the expiratory branch 213a according to the control command, or to control the flow rate or pressure of the exhaled gas od the patient. When specifically implemented, the exhalation controller 214a may include one or more elements that can control flow or pressure, such as an exhalation valve, a check valve, a flow controller, a PEEP (positive expiratory end pressure) valve, etc. The inspiratory controller 214b is arranged on the inspiratory branch 213b to switch on or off the inspiratory branch 213b according to the control command, or to control the flow rate or pressure of the output gas. When specifically implemented, the inspiratory controller 214b may include one or more elements that can control flow or pressure, such as an exhalation valve, a check valve, a flow controller, etc.

The memory 215 may be configured to store data or program, such as data sampled by each sensor, data generated by the processor through calculation, or image frames generated by the processor. The image frames may be 2D or 3D images. Optionally, the memory 215 may store a graphical user interface, one or more default image display settings, and programming instructions for the processor. The memory 215 may be a tangible and non-transient computer-readable medium, such as flash memory, RAM, ROM, EEPROM, and the like.

In some embodiments, the processor 30 is configured to execute instructions or programs, control various control valves in the respiration assist apparatus, the gas source interface 212 and/or the respiratory line, or process the received data, generate the required calculation or determination results, or generate visual data or graphics, and output the visual data or graphics to the display 50 for displaying.

The above are some descriptions of medical device as a ventilator. It should be noted that FIG. 3 above is only an example of a ventilator, which is not configured to limit the ventilator to such a structure.

In some embodiments, the medical device can also be an anesthetic machine, which is mainly configured to provide anesthetic gas, send the anesthetic gas to the respiratory system of the patient through a ventilator, and control the amount of anesthetic gas inhaled. Referring to FIG. 4, the medical device of some embodiments may also include a respiratory interface 311, a gas source interface 312, a respiration assistance apparatus 320, an anesthetic drug output apparatus 330, a respiratory line, and a memory 350.

The gas source interface 312 is configured to connect with the gas source (not shown), which is configured to provide gas. The gas can usually be oxygen, nitrous oxide (laughing gas), air and so on. In some embodiments, the gas source can be a compressed gas cylinder or a central gas supply source, which supplies gas to the anesthetic machine through the gas source interface 312. The types of gas include oxygen $O_2$, nitrous oxide $N_2O$, air, etc. The gas source interface 312 can include conventional components, such as pressure gauge, pressure regulator, flowmeter, pressure-reduction valve and $N_2O$-$O_2$ proportional control protection device, which are respectively configured to control the flow of various gases (such as oxygen, laughing gas and air). The gas, which is inputted from the gas source interface 312, enters the respiratory line and forms a mixed gas with the original gas in the respiratory line.

The respiration assistance apparatus 320 is configured to provide power for the non-spontaneous respiration, maintain the airway unblocked. In some embodiments, the respiration assistance apparatus 320 is connected with the gas source interface 312 and the respiratory line to control the delivery of the gas provided by the external gas source to the patient through the respiratory line. In some specific embodiments, the respiration assistance apparatus 320 mixes the fresh gas which is inputted from the gas source interface 312 with the gas which is exhaled by the patient in the respiratory line and the anesthetic drug which is outputted from the anesthetic drug output apparatus 330, and outputs the mixture to the respiratory interface 311 through the inspiratory branch 340b to drive the patient to inhale, and receives the exhaled gas from the patient through the expiratory branch 340a. In a specific embodiment, the respiration assistance apparatus 320 usually includes a machine-controlled ventilation module, and the gas duct of the machine-controlled ventilation module is connected with the respiratory line. During the anesthesia maintenance stage of the surgery or when the patient does not resume spontaneous respiration, the machine-controlled ventilation module is configured to provide the patient with respiratory power. In some embodiments, the respiration assistance apparatus 320 also includes a manual ventilation module, and the gas duct of the manual ventilation module is connected with the respiratory line. During the induction stage before intubation, the manual ventilation module is usually configured to assist the patient in respiration. When the respiration assistance apparatus 320 includes both machine-controlled ventilation module and manual ventilation module, the machine-controlled ventilation mode and the manual ventilation mode can be switched through a machine-controlled or manual switch (such as a three-way valve), so as to connect the machine-controlled ventilation module or manual ventilation module with the respiratory line for controlling the respiration of the patient. Those skilled in the art should understand that the anesthetic machine can only include a machine-controlled ventilation module or a manual ventilation module according to specific requirements.

The anesthetic drug output apparatus 330 is configured to provide anesthetic drugs. Generally, the anesthetic drugs are mixed into fresh air which is guided by the gas source interface 312 in the form of gas and delivered to the respiratory line together. In a specific embodiment, the anesthetic drug output apparatus 330 is realized by using an anesthetic drug volatilization tank. Anesthetic drugs are usually liquid and stored in the anesthetic drug volatilization tank. Optionally, the anesthetic drug volatilization tank can include a heating device to heat the anesthetic drug to volatilize and generate anesthetic steam. The anesthetic drug output apparatus 330 is connected with the pipeline of the gas source interface 312. The anesthetic steam is mixed with the fresh air which is guided from the gas source interface 312 and then transmitted to the respiratory line together.

In some embodiments, the respiratory line includes an inspiratory branch 340b, an expiratory branch 340a and a soda-lime tank 340c, wherein the inspiratory branch 340b and the expiratory branch 340a are connected to form a closed loop. The soda-lime tank 340c is arranged at expiratory branch 340a. The mixed gas of anesthetic steam and fresh air which is guided from the gas source interface 312, is inputted from an inlet of the inspiratory branch 340b and supplied to the patient 20 through the respiratory interface 311 which arranged at an outlet of the inspiratory branch 340b. The respiratory interface 311 may be a mask, a nasal intubation, or an endotracheal intubation. In a preferred embodiment, the inspiratory branch 340b is provided with a check valve, which opens in the inspiratory phase and closes in the expiratory phase. The expiratory branch 340a is also provided with a check valve, which is closed in the inspiratory phase and opened in the expiratory phase. The inlet of the expiratory branch 340a is communicated with the respiratory interface 311. When the patient exhales, the exhaled gas enters the soda-lime tank 340c through the expiratory branch 340a. The carbon dioxide in the exhaled gas is filtered by the substances in the soda-lime tank 340c, and the filtered gas is recycled into the inspiratory branch 340b.

The memory 350 may be configured to store data or program, such as data sampled by each sensor, data generated by the processor through calculation, or image frames generated by the processor. The image frames may be 2D or 3D images. Optionally, the memory 350 may store a graphical user interface, one or more default image display settings, and programming instructions for the processor. The memory 350 may be a tangible and non-transient computer-readable medium, such as flash memory, RAM, ROM, EEPROM, and the like.

In some embodiments, the processor 30 is configured to execute instructions or programs, control various control valves in the respiration assistance apparatus 320, the gas source interface 310 and/or the respiratory line, or process the received data, generate the required calculation or determination results, or generate visual data or graphics, and output the visual data or graphics to the display 50 for displaying.

The above are some descriptions of medical device as an anesthetic machine. It should be noted that FIG. 4 above is only an example of anesthetic machine, which is not configured to limit the anesthetic machine to such a structure.

The above describes some examples in which the medical device is a patient monitor, a patient monitoring module, and a medical ventilation apparatus, such as a ventilator or an anesthetic machine. The medical device of this disclosure can acquire the respiratory parameters of the patient through the sensor 10 and process them through the processor 30. The display 50 visually displays the processed results, which will be described in detail below.

The sensor 10 is configured to acquire at least one respiratory parameter of the patient, such as a gas flow rate of the patient during the ventilation process, and further as, a pressure of the patient during the ventilation process, which pressure reflects the pressures acting on different points of the respiratory system of the patient during the ventilation process, such as one or more of airway pressure, intrathoracic pressure, carinal pressure, intrapulmonary pressure, esophageal pressure and intragastric pressure. These are described below.

In some embodiments, the sensor 10 may be a flow sensor for acquiring the gas flow rate of the patient during the ventilation process. In some embodiments, the gas flow rate of the patient during the ventilation process includes at least inspiratory flow rate of the patient. In some embodiments, the sensor 10 may be a flow sensor arranged at the patient end, such as a flow sensor arranged at the patient interface, and the gas flow rate is the gas flow rate which is acquired by the flow sensor during inspiration. In some embodiments, there are multiple flow sensors, which includes an inspiratory flow sensor and an expiratory flow sensor which are arranged at the mechanical ventilation end. For example, for a ventilator, the sensor 10 may be an inspiratory flow sensor arranged at the inspiratory branch 213b and an expiratory flow sensor arranged at the expiratory branch 213a. For an anesthetic machine, the sensor 10 may be an inspiratory flow sensor arranged at the inspiratory branch 340b and an expiratory flow sensor arranged at the expiratory branch 340a. The gas flow rate is the difference between the flow rates acquired by the inspiratory flow sensor and the expiratory flow sensor during the inspiratory period. In some embodiments, the flow sensor can also be a Y-piece flow sensor, which directly measures the inflow and outflow flow rates at the patient end as the gas flow rate. Of course, the energy acting on the respiratory system of the patient during the mechanical ventilation process can be calculated by the gas flow rate during the whole respiratory period, including the gas flow rate during inhalation and exhalation.

In some embodiments, the sensor 10 may be one or more pressure sensors 10. The pressure sensor 10 is configured to acquire the pressure of the patient during the ventilation process, which pressure reflects the pressures acting on different points of the respiratory system of the patient during the ventilation process, such as one or more of airway pressure, intrathoracic pressure, carinal pressure, intrapulmonary pressure, esophageal pressure and intragastric pressure.

In some embodiments, the sensor 10 may be a pressure sensor, such as a catheter-type pressure sensor or an optical fiber-type pressure sensor. By inserting the pressure sensor to the corresponding site of the respiratory system of the patient, the pressure at the corresponding site can be acquired. For example, if the pressure sensor is inserted into the airway of the patient, the airway pressure can be acquired. If the pressure sensor is inserted into the esophagus, the esophageal pressure can be acquired. If the pressure sensor is inserted into the stomach, the intragastric pressure can be acquired. If the pressure sensor is inserted into the carina inside the trachea, the carinal pressure can be acquired. If the pressure sensor is inserted into the stomach, the intragastric pressure can be acquired. If the pressure sensor is inserted into the thoracic cavity through a wound incision, the intrathoracic pressure can be acquired. Of course, the esophageal pressure can also be used to approximately replace intrathoracic pressure. In some embodiments, the pressures at some points in the respiratory system can also be used to replace or calculate the pressure at other points, which is illustrated by several examples below.

In some embodiments, carinal pressure may be used to replace intrapulmonary pressure. In some embodiments, esophageal pressure may be used to replace intrathoracic pressure. In some embodiments, intragastric pressure may be used to replace intraabdominal pressure.

In some embodiments, the processor 30 may calculate intrapulmonary pressure according to the airway pressure. For example, in some embodiments, the processor 30 calculates the intrapulmonary pressure according to the airway pressure, a real-time volume, and the gas flow rate described above. In a specific example, it can be calculated by the following formula:

$$\mathrm{Plung(t)} = \frac{V(t)*[Paw(t)-PEEP]}{V(t)+\tau*Flow(t)}.$$

Wherein Plung(t) refers to a function of intrapulmonary pressure which changes with time t, or refers to a real-time intrapulmonary pressure. $V(t)$ refers to a function of the volume which changes with time t, or refers to the real-time volume. $Paw(t)$ refers to a function of airway pressure which changes with time t, or refers to a real-time airway pressure. $Flow(t)$ refers to a function of the gas flow rate of the patient during the ventilation process which changes with time t, or refers to a real-time gas flow rate of the patient during the ventilation process. PEEP refers to a positive expiratory end pressure with a unit of cmH2O. $\tau$ is a time constant, which can be calculated by the following formula:

$$\tau = \frac{t}{\ln[V(0)/V(t)]}.$$

In some embodiments, the processor 30 may calculate transpulmonary pressure by subtracting either the esophageal pressure or the intrathoracic pressure from either the intrapulmonary pressure or the airway pressure. For example, the transpulmonary pressure is obtained by subtracting the esophageal pressure from the airway pressure. In some embodiments, the processor 30 may also correct the transpulmonary pressure, which is described in detail below.

In some embodiments, the processor 30 also corrects the transpulmonary pressure by the airway pressure value and the esophageal pressure value in the state where the positive expiratory end pressure is zero and non-zero. Specifically, the processor 30 acquires the airway pressure $Paw_{PEEP}$ and esophageal pressure $Pes_{PEEP}$ in a state where the positive expiratory end pressure is non-zero, and acquires the airway pressure $Paw_{ZEEP}$ and esophageal pressure $Pes_{ZEEP}$ in a state where the positive expiratory end pressure is zero. The processor 30 adds the transpulmonary pressure with ($Paw_{PEEP}$-$Paw_{ZEEP}$) and subtracts ($Pes_{PEEP}$-$Pes_{ZEEP}$) from the sum, to obtain the corrected transpulmonary pressure.

In some embodiments, the processor 30 also corrects the transpulmonary pressure value by lung compliance and chest wall compliance. Specifically, the processor 30 acquires the static lung compliance Clung and the static chest wall compliance Ccw. It should be noted that there are many methods for processor 30 to obtain the static lung compliance Clung and the static chest wall compliance Ccw. For example, the processor 30 can obtain the static chest wall compliance Ccw through the following formula:

$$\mathrm{Ccw} = \frac{\mathrm{TV}}{\mathrm{PesI}-\mathrm{PEEP}_{es}}.$$

Wherein, TV is tidal volume, PesI is esophageal pressure at inspiratory end, $PEEP_{es}$ is esophageal pressure at expiratory end. It should be noted that in some embodiments, the tidal volume TV can be obtained by accumulating the gas flow rate.

Then the static total compliance $C_{state}$ can be calculated through the following formula:

$$C_{state} = \frac{\mathrm{TV}}{\mathrm{Pplat}-\mathrm{PEEP}}.$$

Wherein, TV is tidal volume, Pplat is plateau pressure, PEEP is positive expiratory end pressure. It should be noted that, the plateau pressure Pplat can be considered as an inspiratory end pressure, for example, the mean pressure in 100ms before switching the expiration to inspiration.

When the static total compliance $C_{state}$ and the static chest wall compliance Ccw are calculated, the static lung compliance Clung can be calculated by solving the following equation:

$$\frac{1}{\mathrm{Clung}} + \frac{1}{\mathrm{Ccw}} = \frac{1}{C_{state}}.$$

After acquiring the static lung compliance Clung and the static chest wall compliance Ccw, the processor 30 can calculate an error compensation value through the following formula:

$$\Delta \mathrm{Ptrans}_{erro} = \mathrm{Ptrans} - \mathrm{Plung} * \frac{\frac{1}{\mathrm{Clung}}}{\frac{1}{\mathrm{Clung}}+\frac{1}{\mathrm{Ccw}}}.$$

Wherein, $\Delta Ptrans_{erro}$ is the error compensation value, Ptrans is the transpulmonary pressure value, Plung is the intrapulmonary pressure value.

The processor 30 subtracts the error compensation value from the transpulmonary pressure value to obtain the corrected transpulmonary pressure value.

In some embodiments, the processor 30 may calculate a transdiaphragmatic pressure by subtracting either intraabdominal pressure or intragastric pressure from either intrathoracic pressure or esophageal pressure. For example, the transdiaphragmatic pressure can be obtained by subtracting the intragastric pressure from the esophageal pressure. It should be noted that in some embodiments, if the pressure sensor is inserted into the abdomen through a wound incision, the intraabdominal pressure can be acquired. In some embodiments, the processor 30 may also correct the transdiaphragmatic pressure. For example, the processor 30 acquires the esophageal pressure $Pes_{PEEP}$ and the intragastric pressure $Psto_{PEEP}$ when the positive expiratory end pressure is non-zero, and acquires the esophageal pressure $Pes_{ZEEP}$ and the intragastric pressure $Psto_{ZEEP}$ when the positive expiratory end pressure is zero. The processor 30 adds the transdiaphragmatic pressure with ($Pes_{PEEP}$-$Pes_{ZEEP}$) and subtracts ($Psto_{PEEP}$-$Psto_{ZEEP}$) from the sum, to obtain the corrected transdiaphragmatic pressure.

The above are some descriptions of the airway pressure, intrathoracic pressure, carinal pressure, intrapulmonary pressure, esophageal pressure, intragastric pressure, intraabdominal pressure, transpulmonary pressure and transdiaphragmatic pressure.

It can be seen from the above description that in some embodiments, the sensor 10 can include multiple sensors 10 which include pressure sensors and flow sensors. Therefore, in some embodiments, the respiratory parameters of the patient that can be acquired by the medical device through the sensor 10 are the gas flow rate of the patient during the ventilation process and the pressure of the patient during the ventilation process.

In some embodiments, the processor 30 acquires a target of a ventilation control parameter and a ventilation treatment strategy, and calculates a target of at least one associated control parameter according to the target of the ventilation control parameter and the ventilation treatment strategy. The following describes how the processor 30 acquires the target of the ventilation control parameter and the ventilation treatment strategy.

In some embodiments, the processor 30 may determine the target of the ventilation control parameter in response to a received instruction therefor. In some embodiments, the processor 30 determines the target of the ventilation control parameter according to one or more of respiratory parameter or patient information. The patient information can be gender, age and past medical history of the patient.

In some embodiments, the processor 30 determines the ventilation treatment strategy in response to a received instruction therefor. In some embodiments, the processor 30 determines the ventilation treatment strategy according to one or more of respiratory parameter or patient information. In some embodiments, the ventilation treatment strategy includes a minimum respiratory power strategy.

It can be seen that the processor 30 can automatically set the target of the ventilation control parameter and obtain the ventilation treatment strategy according to one or more of the respiratory parameters or patient information, and can also set the target of the ventilation control parameter and obtain the ventilation treatment strategy according to a user input. Therefore, in some embodiments, the respiratory state of the patient can be displayed so that the user can issue an instruction for the target of the ventilation control parameter or an instruction for the ventilation treatment strategy according to the displayed respiratory state of the patient. Specifically, in some embodiments, the processor 30 generates the respiratory state of the patient according to the respiratory parameters, and the display 50 displays the generated respiratory state of the patient. In some embodiments, when determining that the respiratory state of the patient changes according to the respiratory parameters, the processor 30 generates indication information for indication. Further, the doctor can reformulate a new target of the ventilation control parameter or a new ventilation treatment strategy according to the indication information, or the medical ventilation device can automatically trigger the change of the target of the ventilation control parameter or the ventilation treatment strategy according to the indication information when the respiratory parameters change. In this disclosure, the respiratory state is generated according to the respiratory parameter. As mentioned above, the respiratory parameter can be the gas flow rate of the patient during the ventilation process and the pressure of the patient during the ventilation process. According to these two, the compliance and time constant of the patient can be calculated as the respiratory state of the patient. The compliance here can refer to the total compliance of the patient, which includes the static total compliance and/or dynamic total compliance. The compliance here can also refer to the lung compliance of the patient, including static lung compliance and/or dynamic lung compliance. In some embodiments, the dynamic total compliance and time constant of the patient are calculated and displayed as the respiratory state of the patient. When a respiratory state of the patient changes, it can mean that his respiratory state changes from one state to another. For example, taking the compliance of the patient as an example, the change of the can mean that the value of compliance in one range changes to a value in another range.

The above is a description about that the processor 30 acquires the target of the ventilation control parameter and acquires the ventilation treatment strategy. In some embodiments, the processor 30 calculates the target of the at least one associated control parameter according to the target of the ventilation control parameter and the ventilation treatment strategy. In some embodiments, the processor 30 also calculates recommended ranges of the ventilation control parameter and the at least one associated control parameter.

In some embodiments, the processor 30 obtains a ventilation parameter which characterizes a ventilation state of the patient according to the acquired respiratory parameter. In some embodiments, the ventilation parameter includes one or more of the ventilation control parameter or the associated control parameter. Specifically, the ventilation control parameter may include ventilation volume per minute, the associated control parameter may include a tidal volume and/or a respiratory rate.

In some embodiments, the processor 30 generates and outputs a visual graph according to the ventilation parameter of the patient, the target of the ventilation control parameter and the target of the at least one associated control parameter, wherein the ventilation state of the patient, the target of the ventilation control parameter and the target of the at least one associated control parameter are displayed through the visual graph. The display 50 displays the content generated by the processor 30, such as the above visual graph.

The following may be explained by the adaptive assisted ventilation mode which is developed from the mandatory minute ventilation mode. In the adaptive assisted ventilation mode of some embodiments, the ventilation control parameter may be ventilation volume per minute, the associated control parameter may include tidal volume and respiratory rate, and the respiratory state may include compliance of the patient and time constant.

Firstly, the target of the ventilation volume per minute, which can be preset by the user or automatically set by the device, and the ventilation treatment strategy, such as the minimum respiratory power strategy, are set. Then the target of the tidal volume and the target of the respiratory rate are calculated according to the target of the ventilation volume per minute and the minimum respiratory power strategy. Specifically, since the respiratory power of the patient can be calculated by the compliance of the patient the and tidal volume, and the ventilation volume per minute can also be calculated by the tidal volume and respiratory rate of the patient, when the target of the ventilation volume per minute is known, the corresponding values of the tidal volume and the respiratory rate, under the constraint of the minimum respiratory power, can be solved as the target of the tidal volume and the target of the respiratory rate respectively. Therefore, when the current values of the ventilation parameters of the patient, such as ventilation volume per minute, tidal volume and respiratory rate, are the target of the ventilation volume per minute, the target of the tidal volume and the target of the respiratory rate respectively, it means that the patient is ventilated in the state of minimum respiratory power at this time.

This patient ventilation mode of patient in the state of minimum respiratory power is essentially a kind of lung protective ventilation, which allows the patient to match better tidal volume and respiratory rate under the expected ventilation volume per minute, so as to achieve or approach and maintain the ventilation treatment purpose of minimum respiratory power in real time to the greatest extent.

In this ventilation mode, the apparatus dynamically monitors the respiratory state of the patient in real time, such as dynamic compliance and time constant, according to the set target of the ventilation volume per minute, and then calculates and matches the target of the tidal volume and the target of the respiratory rate which are required by the patient in real time, and then implements the ventilation control in real time, so as to achieve or approach and maintain the patient in the ventilation state of minimum respiratory power in real time to the greatest extent. Therefore, in this ventilation mode, the user is very concerned about the tidal volume, respiratory rate, ventilation volume per minute, and even dynamic compliance and time constant of the patient which are actually controlled by the apparatus, so that the user can visually determine whether the respiratory state and ventilation state of the patient are within the recommended range, and determine whether a change trend in the ventilation process conforms to the expected treatment purpose, decide whether it is necessary to timely adjust the strategy according to the respiratory state and ventilation state of the patient when necessary, such as changing the ventilation control parameters and basically switching the ventilation mode, and so on.

Therefore, in some embodiments, the processor 30 generates and outputs a visual graph based on the ventilation parameter of the patient, the target of the ventilation control parameter and the target of the at least one associated control parameter, and displays the ventilation state of the patient, the target of the ventilation control parameter and the target of the at least one associated control parameter through the visual graph. The display 50 displays the content generated by the processor 30, such as the above visual graph. In some specific embodiments, the visual graph displays the ventilation state of the patient by displaying at least one of the current value and change trend of the ventilation parameter. In other embodiments, the processor 30 calculates the recommended range of the ventilation control parameter and at least one associated control parameter, and displays them in the display 50 through the visual graph. In some embodiments, each ventilation parameter has a corresponding visual graph. Therefore, the visual graph also has the parameter name of the corresponding ventilation parameter. Preferably, it can also have the unit name of the corresponding ventilation parameter.

There are many implementation ways for the visual graph, such as arc graph, histogram, two-dimensional curve and altimeter graph which are with time as horizontal and vertical axis, which are illustrated by examples below.

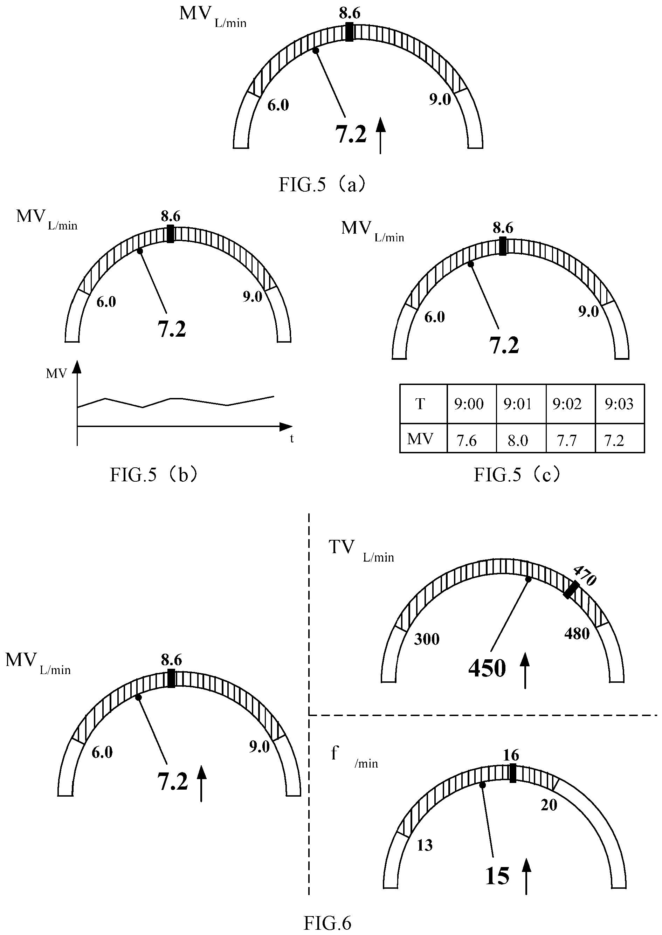

Referring to FIG. 5, in some embodiments, the visual graph includes an arc graph, wherein the arc graph includes an arc which represents a value range and a first indicator which represents a current value of a corresponding ventilation parameter, such as a pointer. In some embodiments, a number, which represents the current value of the corresponding ventilation parameter, is also arranged adjacent to the first indicator. In some embodiments, the arc also has a number which represents a value corresponding to a position on the arc. In some embodiments, the arc includes a safety segment which represents a recommended range of the corresponding ventilation parameter, wherein the safety segment is displayed in a manner which is different from other segment(s) of the arc, for example, the safety segment and other segment(s) are in different colors. In some embodiments, the arc graph further comprises a second indicator which represents a target of the corresponding ventilation parameter. Preferably, a number which represents a value of the target of the corresponding ventilation parameter, is provided adjacent to the second indicator. In some embodiments, the arc graph further comprises a third indicator which represents a change trend of the corresponding ventilation parameter, and the third indicator is adjacent to the number which represents the current value of the corresponding ventilation parameter. Alternatively, a two-dimensional curve or a numerical table which represents the change trend of the corresponding ventilation parameter is also arranged adjacent to the arc graph. In the example of FIG.5, the semicircular arc segment is an arc graph, MV represents ventilation volume per minute, that is, the name of the ventilation parameter which corresponds to the arc graph. L/min, which is adjacent to MV, represents the unit, the values 6.0 and 9.0 at the semicircular arc segment represent the value of MV at the corresponding position on the arc segment, the arc segment which is filled with lines is the safety segment, and the arc segment is also arranged with the second indicator (that is, the black filled scale in the figure), which represents the target of the ventilation volume per minute, and its annex is also provided with a value which represents the target of the ventilation volume per minute, namely, 8.6 in the figure. 7.2 represents the current value of MV and points to the corresponding position at the arc segment through the first indicator, that is, the pointer in the figure. In FIG. 5 (a), the right side of 7.2 is provided with an upward arrow, that is, the third indicator. When the arrow is upward, it means that the change trend of the ventilation volume per minute is getting greater, on the contrary, means that the change trend of the ventilation volume per minute is getting lower. In FIG. 5 (b), a two-dimensional curve showing the change trend of ventilation volume per minute is displayed below the arc graph for the user to view. In FIG. 5 (c), a numerical table showing the change trend of ventilation volume per minute is displayed at the bottom of the arc graph for the user to view. FIG. 6 is an example which simultaneously displays visual graphs which are corresponding to ventilation volume per minute, tidal volume, and respiratory rate, wherein TV refers to the tidal volume and f refers to the respiratory rate.

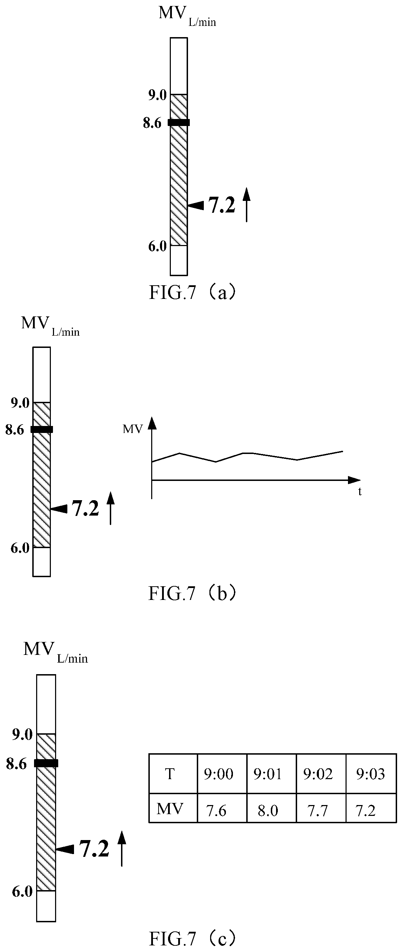

Please refer to FIG. 7. In some embodiments, the visual graph includes a histogram, including a columnar segment which represents a value range and a fourth indicator which represents a current value of a corresponding ventilation parameter.

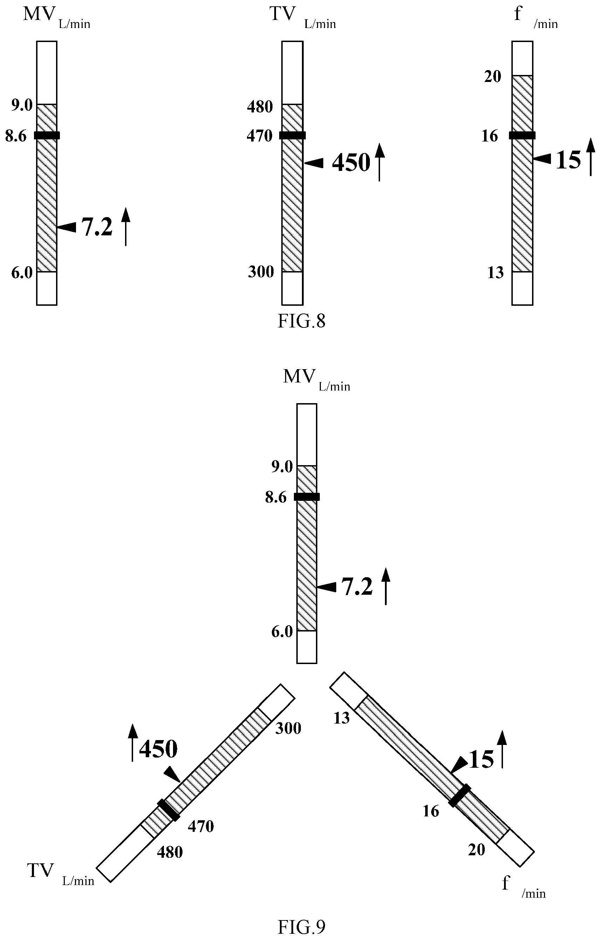

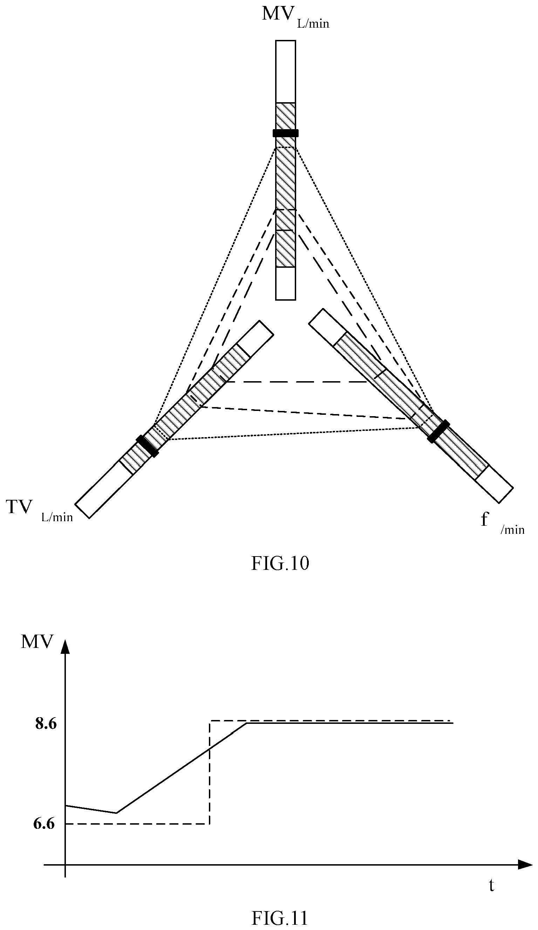

In some embodiments, a number, which represents the current value of the corresponding ventilation parameter, is arranged adjacent to the fourth indicator. In some embodiments, the columnar segment comprises a number which represents a value corresponding to a position on the columnar segment. In some embodiments, the columnar segment includes a safety segment which represents a recommended range of the corresponding ventilation parameter, wherein the safety segment is displayed in a manner which is different from other segment(s) of the columnar segment, for example, the safety segment and other segment(s) are in different colors. In some embodiments, the histogram further comprises a fifth indicator which represents the target of the corresponding ventilation parameter. Preferably, a number which represents a value of the target of the corresponding ventilation parameter is provided adjacent to the fifth indicator. In some embodiments, the histogram further comprises a sixth indicator which represents a change trend of the corresponding ventilation parameter, wherein the sixth indicator is adjacent to the number which represents the current value of the corresponding ventilation parameter. Alternatively, a two-dimensional curve or a numerical table which represents a change trend of the corresponding ventilation parameter is arranged adjacent to the histogram. In the example in FIG. 7, MV represents the ventilation volume per minute, that is, the name of the ventilation parameter which corresponds to the histogram, L/min, which is adjacent to MV, represents the unit. The values 6.0, 8.6 and 9.0 on the left of the vertical columnar segment respectively, represent values of MV at the corresponding positions. The columnar segment which is filled with lines in the figure is the safe segment. The columnar segment is also provided with the fifth indicator, namely, the black filled scale in the figure, which represents the target of the ventilation volume per minute, and its annex is also provided with a value which represents the target of the ventilation volume per minute, namely, 8.6 in the figure. 7.2 represents the current value of MV, and points to the corresponding position on the columnar segment through the fourth indicator, that is, the triangle head filled with black in the figure. In FIG. 6 (a), the right side of 7.2 is provided with an upward arrow, that is, the third indicator. When the arrow is upward, it means that the change trend of the ventilation volume per minute is getting greater, on the contrary, means that the change trend of the ventilation volume per minute is getting lower. In FIG. 6 (b), a two-dimensional curve showing the change trend of the ventilation volume per minute is displayed at the right side of the histogram for the user to view. In FIG. 6 (c), a numerical table showing the change trend of ventilation volume per minute is displayed at the right side of the histogram for the user to view. FIG. 8 is an example which simultaneously displays visual graphs which are corresponding to ventilation volume per minute, tidal volume, and respiratory rate, wherein TV refers to the tidal volume and f refers to the respiratory rate. In some embodiments, the histograms which correspond to different ventilation parameters are distributed in a rotationally symmetric manner or side by side. FIG. 8 is an example of side-by-side distribution of histograms which correspond to each ventilation parameter. FIG. 9 is an example of rotationally symmetric distribution of the histograms which correspond to each ventilation parameter. In other embodiments, the values of different ventilation parameters on the corresponding histogram at the same time are connected to form line segments. The line segments at different times are displayed in different ways, for example, the line segment at the current time is displayed in a first color, the line segments at the previous times are displayed in a second color in different brightness. For example, the line segment at the current time is displayed in blue, and the line segment at the previous time is displayed in gray. The darker the brightness of gray, the closer to the current time. FIG. 10 is an example, in which the positions at the column section, where is engraved with lines, on each histogram in the figure, is the fourth indicator. The values of the ventilation volume per minute, tidal volume and respiratory rate on the corresponding histogram at the same time, are connected to form line segments. The densest imaginary line segments in the figure represent the line segments at the current time, the second densest imaginary line segments represent the line segments at the previous one minute, and the third densest imaginary line segments represent the line segment at the previous two minutes.

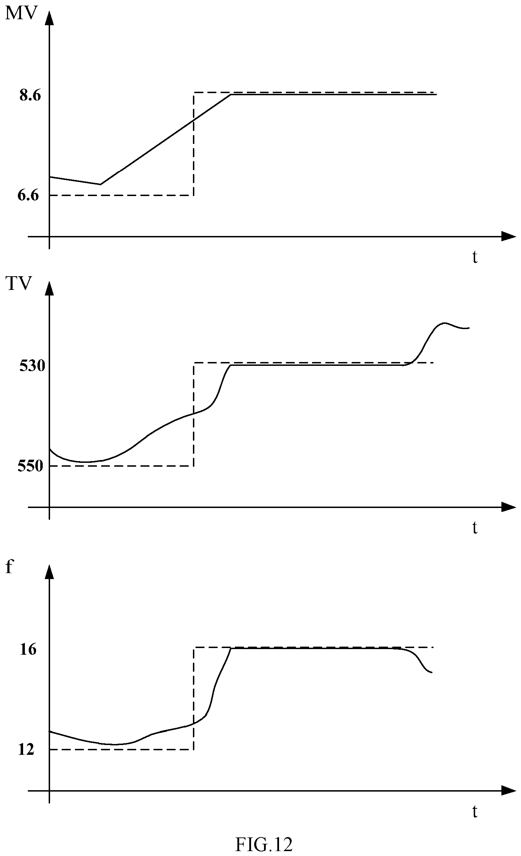

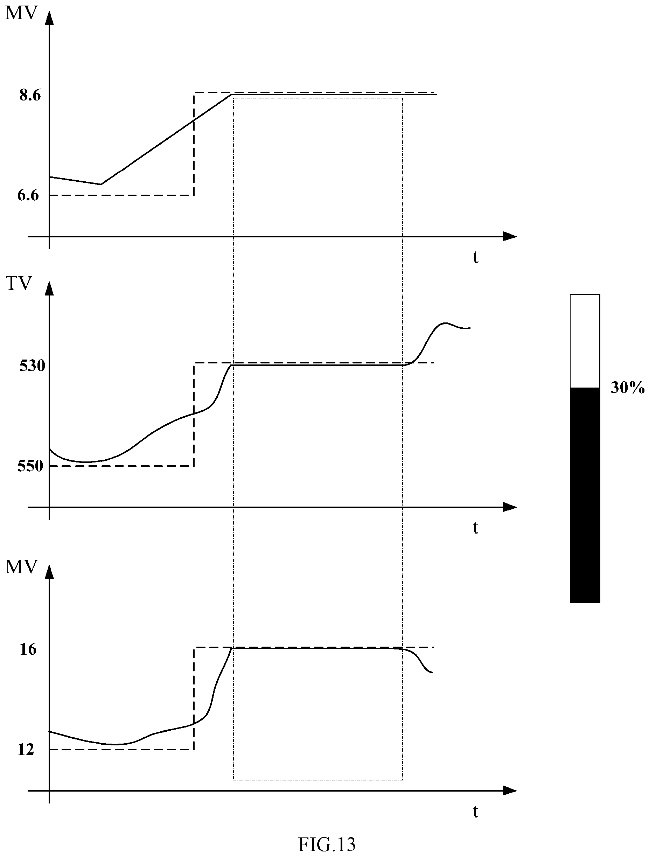

Please refer to FIG. 11. In some embodiments, the visual graph includes a two-dimensional curve. The horizontal coordinate of the two-dimensional curve is time, and the vertical coordinate of the two-dimensional curve is the corresponding ventilation parameter. The two-dimensional curve comprises a first curve which is generated by current values of corresponding ventilation parameter at different times. This means that each ventilation parameter corresponds to a two-dimensional curve. In some embodiments, the two-dimensional curve further comprises a second curve, which is generated by the targets of the ventilation parameters that correspond to the ventilation parameters at different times. In some embodiments, the second curve is displayed in a manner which is different from the first curve, for example, the color of the first curve is black, and the color of the second curve is gray. FIG. 11 is an example, in which MV refers to the ventilation volume per minute, the first curve is a real line segment, and the second curve is an imaginary line segment. FIG. 12 is an example which simultaneously displays visual graphs which are corresponding to ventilation volume per minute, tidal volume, and respiratory rate, wherein TV refers to the tidal volume and f refers to the respiratory rate. In some embodiments, when the processor 30 determines that the patient has reached the minimum respiratory power state, the processor 30 further generates an area which indicates that the patient has reached the minimum respiratory power state on the two-dimensional curve, and the display 50 displays it accordingly. For example, the area, which indicates that the patient has reached the minimum respiratory power state, is displayed on the two-dimensional curve. For example, the area surrounded by a rectangle of dotted lines in FIG. 13, represents the area which indicates the patient has reached the minimum respiratory power state. In some embodiments, the processor 30 also calculates and controls the display 50 to display a time proportion within a preset time period for the patient to reach the minimum respiratory power state. For example, in the right column segment in FIG. 13, 30% represents the time proportion for the patient to reach the minimum respiratory power state from the start time to the current time.

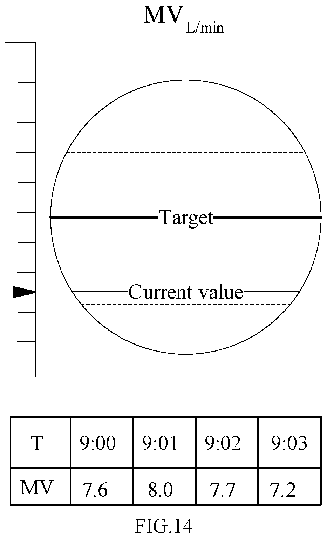

Referring to FIG. 14, in some embodiments, the visual graph includes an altimeter graph which includes a line segment which is vertical and scaled, and a circle. The circle includes a seventh indicator which represents a current value of a corresponding ventilation parameter, wherein the seventh indicator is a horizontal subtense in the circle. The scale of the line segment, which corresponds to the subtense, represents the current value of the corresponding ventilation parameter. In some embodiments, the circle also includes a recommended area that represents the recommended range of the corresponding ventilation parameter. The recommended area is displayed in a manner which is different from other area(s) of the circle, for example, the recommended area is filled with gray, and other areas are white. In some embodiments, the circle further comprises an eighth indicator which represents the target of the corresponding ventilation parameter. The eighth indicator is the horizontal diameter of the circle, and the height of the circle is arranged so that the horizontal diameter which corresponds to the scale of the line segment is the target of the corresponding ventilation parameter. In some embodiments, a two-dimensional curve or a numerical table which represents the change trend of the corresponding ventilation parameter is also arranged adjacent to the altimeter graph. FIG. 14 is an example. The range between the subsenses of the two dotted lines in the circle is the recommended area.

These are some descriptions of medical devices.

In some embodiments of this disclosure, a ventilation information display method for a medical ventilation device is also provided. The medical ventilation device provides respiratory support for a patient. The medical ventilation device can be a ventilator or anesthetic machine disclosed herein.

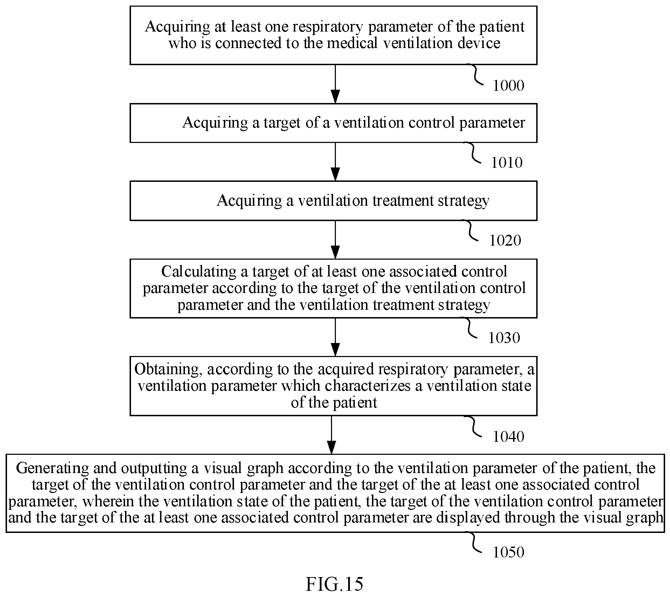

Referring to FIG. 15, in some embodiments, the ventilation information display method includes the following steps.

In step 1000, at least one respiratory parameter of a patient who is connected to the medical ventilation device, is acquired. The respiratory parameter can be acquired by the sensor 10 disclosed above, and can include, for example, a gas flow rate of the patient during the ventilation process, and further as, a pressure of the patient during the ventilation process, which pressure reflects the pressures acting on different points of a respiratory system of the patient during the ventilation process, such as one or more of airway pressure, intrathoracic pressure, carinal pressure, intrapulmonary pressure, esophageal pressure and intragastric pressure. For further explanation, please refer to the above description, which is not repeated here.

In step 1010, a target of a ventilation control parameter, is acquired.

In some embodiments, step 1010 may include determining the target of the ventilation control parameter in response to a received instruction therefor. In some embodiments, step 1010 may include determining the target of the ventilation control parameter according to one or more of respiratory parameter or patient information. The patient information can be gender, age and past medical history of the patient.

In step 1020, a ventilation treatment strategy is acquired.

In some embodiments, step 1020 may include determining the ventilation treatment strategy in response to a received instruction therefor. In some embodiments, step 1020 may include determining the ventilation treatment strategy according to one or more of respiratory parameter or patient information. In some embodiments, the ventilation treatment strategy includes a minimum respiratory power strategy.

It can be seen that, the target of the ventilation control parameter can be automatically set according to one or more of the respiratory parameters or patient information, and the ventilation treatment strategy can also be obtained according to one or more of the respiratory parameters or patient information. Meanwhile, the target of the ventilation control parameter can be set according to a user input, and the ventilation treatment strategy can also be obtained according to a user input. Therefore, in some embodiments, a respiratory state of the patient can be displayed so that the user can issue an instruction for the target of the ventilation control parameter or an instruction for the ventilation treatment strategy according to the displayed respiratory state of the patient. Specifically, in some embodiments, the method further includes a step of generating a respiratory state of the patient according to the respiratory parameter and displaying the generated respiratory state of the patient. In some embodiments, the method further includes a step of generating indication information for indication when determining that the respiratory state of the patient changes according to the respiratory parameter. Further, the doctor can reformulate a new target of the ventilation control parameter or a new ventilation treatment strategy according to the indication information, or the medical ventilation device can automatically trigger the change of the target of the ventilation control parameter and/or the ventilation treatment strategy according to the indication information when the respiratory parameters change. In this disclosure, the respiratory state is generated according to the respiratory parameter. As mentioned above, the respiratory parameter can be the gas flow rate of the patient during the ventilation process and the pressure of the patient during the ventilation process. According to these two, the compliance and time constant of the patient can be calculated as the respiratory state of the patient. The compliance here can refer to the total compliance of the patient, which includes the static total compliance and/or dynamic total compliance. The compliance here can also refer to the lung compliance of the patient, including static lung compliance and/or dynamic lung compliance. In some embodiments, the dynamic total compliance and time constant of the patient are calculated and displayed as the respiratory state of the patient. When a respiratory state of the patient changes, it can mean that his respiratory state changes from one state to another. For example, taking the compliance of the patient as an example, the change of the can mean that the value of compliance in one range changes to a value in another range.

In step 1030, a target of at least one associated control parameter is calculated according to the target of the ventilation control parameter and the ventilation treatment strategy. In some embodiments, step 1030 also includes calculating a recommended range of the ventilation control parameter and a recommended range of the at least one associated control parameter.

In step1040, a ventilation parameter which characterizes a ventilation state of the patient is obtained according to the acquired respiratory parameter. In some embodiments, the ventilation parameter include one or more of the ventilation control parameter or the associated control parameter. Specifically, the ventilation control parameter may include ventilation volume per minute, the associated control parameters may include a tidal volume and/or a respiratory rate.

In step1050, a visual graph is generated according to the ventilation parameter of the patient, the target of the ventilation control parameter and the target of the at least one associated control parameter, and then outputted. The ventilation state of the patient, the target of the ventilation control parameter and the target of the at least one associated control parameter are displayed through the visual graph. In some specific embodiments, the visual graph displays the ventilation state of the patient by displaying at least one of the current value and change trend of the ventilation parameter. In other embodiments, the recommended range of the ventilation control parameter and at least one associated control parameter are displayed by the visual graph. In some embodiments, each ventilation parameter has a corresponding visual graph. Therefore, the visual graph also has the parameter name of the corresponding ventilation parameter. Preferably, it can also have the unit name of the corresponding ventilation parameter. There are many implementation ways for the visual graph, such as arc graph, histogram, two-dimensional curve and altimeter graph which are with time as horizontal axis, which are fully described above and not repeated here.

Various exemplary embodiments are described herein. However, those skilled in the art recognizes that changes and modifications may be made to the exemplary embodiments without departing from the scope of this disclosure. For example, the various operation steps and the components configured to perform the operation steps can be implemented in different ways according to the specific application or considering any figure of cost functions associated with the operation of the system (for example, one or more steps can be deleted, modified or combined into other steps).

In addition, as understood by those skilled in the art, the principles herein can be reflected in a computer program product on a computer-readable storage medium which is pre-loaded with computer-readable program code. Any tangible, non-transitory computer-readable storage medium can be used, including magnetic storage devices (hard disk, floppy disk, etc.), optical storage devices (CD-ROM, DVD, Blu ray disk, etc.), flash memory and/or the likes. Computer program instructions can be loaded onto a general-purpose computer, a special-purpose computer, or other programmable data processing device to form a machine, so that these instructions executed on a computer or other programmable data processing device can generate a device to realize a specified function. These computer program instructions may also be stored in a computer-readable memory, which may instruct a computer or other programmable data processing device to operate in a specific manner, so that the instructions stored in the computer-readable memory can form a manufactured article, including an implementation device for realizing a specified function. Computer program instructions can also be loaded on a computer or other programmable data processing device, so that a series of operation steps are performed on the computer or other programmable device to generate a computer implemented process, so that the instructions executed on the computer or other programmable device can provide steps for realizing the specified functions.

Although the principles herein have been shown in various embodiments, many modifications of structures, arrangements, proportions, elements, materials, and components that are particularly applicable to specific environmental and operational requirements may be used without departing from the principles and scope of the present disclosure. The above modifications and other changes or amendments are included in the scope of this disclosure.

The foregoing specific description has been described with reference to various embodiments. However, those skilled in the art can recognize that various amendments and changes may be made without departing from the scope of this disclosure. Accordingly, consideration of this disclosure is illustrative rather than restrictive, and all such modifications are included within its scope. Similarly, there are solutions to the advantages, other advantages, and problems of the various embodiments as described above. However, the benefits, advantages, solutions to problems and any solution that can produce these elements or make them more explicit should not be interpreted as critical, necessary or necessary. The term "include" and any other variations thereof as used herein are non-exclusive inclusions, so that a process, method, article or device that includes a list of elements includes not only these elements, but also other elements that are not explicitly listed or do not belong to the process, method, system, article or device. In addition, the term "connection" and any other variation thereof as used herein refer to physical connection, electrical connection, magnetic connection, optical connection, communication connection, functional connection and/or any other connection.

Those skilled in the art recognize that many changes can be made to the details of the above embodiments without departing from the basic principles of this disclosure. Therefore, the scope of this disclosure shall be determined according to the following attached claims.

## Claims

1. A medical device, comprising a ventilation information display apparatus which comprises:

    a display (50);
    one or more sensors (10), which are configured to acquire at least one respiratory parameter of a patient; and
    a processor (30);
    **characterized in that**, the processor (30) is configured to:

    acquire the at least one respiratory parameter of the patient;
    acquire a target of a ventilation control parameter;
    acquire a ventilation treatment strategy;
    calculate a target of at least one associated control parameter according to the target of the ventilation control parameter and the ventilation treatment strategy;
    obtain, according to the acquired at least one respiratory parameter, a ventilation parameter which characterizes a ventilation state of the patient; and
    generate and output a visual graph according to the ventilation parameter of the patient, the target of the ventilation control parameter and the target of the at least one associated control parameter, wherein the ventilation state of the patient, the target of the ventilation control parameter and the target of the at least one associated control parameter are displayed through the visual graph.

2. The medical device according to claim 1, **characterized in that**,

    the processor (30) is further configured to acquire the target of the ventilation control parameter by: determining the target of the ventilation control parameter in response to a received instruction therefor, or determining the target of the ventilation control parameter according to one or more of the respiratory parameter or patient information; and/or
    the processor (30) is further configured to acquire the ventilation treatment strategy by determining the ventilation treatment strategy in response to a received instruction therefor, or determining the ventilation treatment strategy according to one or more of the respiratory parameter or patient information.

3. The medical device according to claim 2, **characterized in that**, the processor (30) is further configured to:

    generate and display a respiratory state of the patient according to the respiratory parameter; and/or
    when determining that a respiratory state of the patient changes according to the respiratory parameter, generate indication information.

4. The medical device according to claim 1, **characterized in that**, the processor (30) is further configured to:

    calculate a recommended range of the ventilation control parameter and a recommended range of the at least one associated control parameter; and display the recommended range of the ventilation control parameter and the recommended range of the at least one associated control parameter through the visual graph; and/or

the visual graph displays the ventilation state of the patient through displaying at least one of current value and change trend of the ventilation parameter.

5. The medical device according to claim 1 or 4, **characterized in that**, the ventilation parameter comprises one or more of the ventilation control parameter or the associated control parameter.

6. The medical device according to claim 5, **characterized in that**, the visual graph comprises an arc graph, wherein the arc graph comprises an arc which represents a value range and a first indicator which represents a current value of a corresponding ventilation parameter.

7. The medical device according to claim 6, **characterized in that**,

the arc comprises a number which represents a value corresponding to a position on the arc;
the arc comprises a safety segment which represents a recommended range of the corresponding ventilation parameter, wherein the safety segment is displayed in a manner which is different from other segment(s) of the arc;
the arc graph further comprises a second indicator which represents a target of the corresponding ventilation parameter, optionally, a number which represents a value of the target of the corresponding ventilation parameter, is provided adjacent to the second indicator; and/or
a number which represents the current value of the corresponding ventilation parameter, is provided adjacent to the first indicator; wherein the arc graph further comprises a third indicator which represents a change trend of the corresponding ventilation parameter, wherein the third indicator is adjacent to the number which represents the current value of the corresponding ventilation parameter; or a two-dimensional curve or a numerical table which represents a change trend of the corresponding ventilation parameters is arranged adjacent to the arc graph.

8. The medical device according to claim 5, **characterized in that**, the visual graph comprises a histogram comprising a columnar segment which represents a value range and a fourth indicator which represents a current value of a corresponding ventilation parameter.

9. The medical device according to claim 8, **characterized in that**,

the columnar segment comprises a number which represents a value corresponding to a position on the columnar segment;
the columnar segment comprises a safety segment which represents a recommended range of the corresponding ventilation parameter, wherein the safety segment is displayed in a manner which is different from other segment(s) of the columnar segment;
the histogram further comprises a fifth indicator which represents a target of the corresponding ventilation parameter, optionally, a number which represents a value of the target of the corresponding ventilation parameter is provided adjacent to the fifth indicator; and/or
a number which represents the current value of the corresponding ventilation parameter, is provided adjacent to the fourth indicator; wherein the histogram further comprises a sixth indicator which represents a change trend of the corresponding ventilation parameter, wherein the sixth indicator is adjacent to the number which represents the current value of the corresponding ventilation parameter; or a two-dimensional curve or a numerical table which represents a change trend of the corresponding ventilation parameters is arranged adjacent to the histogram.

10. The medical device according to claim 8, **characterized in that**:

the ventilation parameter has multiple ventilation parameters, and the histograms which correspond to different ventilation parameters are distributed in a rotationally symmetric manner or side by side;
wherein the processor (30) is further configured to: connect values of different ventilation parameters on corresponding histograms at a same time to form line segments, and display the line segments at different times in different ways;
wherein the processor (30) displays the line segments at different times in different ways by: displaying a line segment at a current time in a first color, and displaying line segments at previous times in a second color in different brightness.

11. The medical device according to claim 5, **characterized in that**, the visual graph comprises a two-dimensional curve;

wherein a horizontal coordinate of the two-dimensional curve is time, and a vertical coordinate of the two-dimensional curve is a corresponding ventilation parameter;

wherein the two-dimensional curve comprises a first curve, which is generated by current values of the corresponding ventilation parameter at different times and a second curve, which is generated by targets of the ventilation parameters at different times; wherein the second curve is displayed in a manner which is different from the first curve.

12. The medical device according to claim 11, **characterized in that**, the ventilation treatment strategy includes a minimum respiratory power strategy;

when determining that the patient has reached the minimum respiratory power state, an area indicating such, is generated on the two-dimensional curve.

13. The medical device according to claim 4, **characterized in that**, the visual graph comprises an altimeter graph, wherein the altimeter graph comprises a line segment which is vertical and scaled, and a circle; wherein the circle comprises a seventh indicator which represents a current value of a corresponding ventilation parameter, wherein the seventh indicator is a horizontal subtense in the circle, and the scale of the line segment corresponding to the subtense represents a current value of the corresponding ventilation parameter.

14. The medical device according to claim 13, **characterized in that**,

the circle further comprises a recommended area which represents a recommended range of the corresponding ventilation parameter; wherein the recommended area is displayed in a manner which is different from other area(s) of the circle;

the circle further comprises an eighth indicator which represents a target of the corresponding ventilation parameter; wherein the eighth indicator is a horizontal diameter of the circle, and a height of the circle is arranged so that the scale of the line segment, which corresponds to the horizontal diameter, is the target of the corresponding ventilation parameter; and/or

a two-dimensional curve or a numerical table which represents a change trend of the corresponding ventilation parameter is arranged adjacent to the altimeter graph.

**Patentansprüche**

1. Ein medizinisches Gerät mit einer Beatmungsinformationsanzeigevorrichtung, das Folgendes umfasst:

ein Display (50);
einen oder mehrere Sensoren (10), die so konfiguriert sind, dass sie mindestens einen Atemparameter eines Patienten erfassen; und
einen Prozessor (30);
Dadurch charakterisiert, dass der Prozessor (30) für Folgendes konfiguriert ist:

mindestens einen Atemparameter des Patienten erfasst
ein Ziel eines Beatmungssteuerparameters sowie
eine Beatmungsbehandlungsstrategie erfasst;
ein Ziel von mindestens einem zugehörigen Steuerparameter gemäß dem Ziel des Beatmungssteuerparameters und der Beatmungsbehandlungsstrategie berechnet;
gemäß dem erfassten mindestens einen Atemparameter einen Beatmungsparameter erhält, der einen Beatmungszustand des Patienten darstellt; und
ein visuelles Diagramm gemäß dem Beatmungsparameter des Patienten, dem Ziel des Beatmungssteuerparameters und dem Ziel des mindestens einen zugehörigen Steuerparameters erzeugt und ausgibt, wobei der Beatmungszustand des Patienten, das Ziel des Beatmungssteuerparameters und das Ziel des mindestens einen zugehörigen Steuerparameters anhand des visuellen Diagramms angezeigt werden.

2. Das medizinische Gerät nach Anspruch 1, mit der Eigenschaft,
dass der Prozessor (30) ferner konfiguriert ist, das Ziel des Beatmungssteuerparameters zu erfassen, indem:

das Ziel des Beatmungssteuerparameters als Antwort auf eine entsprechende empfangene Anweisung bestimmt wird, oder das Ziel des Beatmungssteuerparameters gemäß einem oder mehreren der Atemparameter oder Patienteninformationen bestimmt wird; und/oder

17

der Prozessor (30) ferner so konfiguriert ist, die Beatmungsbehandlungsstrategie zu erfassen, indem: die Beatmungsbehandlungsstrategie als Antwort auf eine entsprechende empfangene Anweisung bestimmt wird, oder die Beatmungsbehandlungsstrategie gemäß einem oder mehreren der Atemparameter oder Patienteninformationen bestimmt wird.

3. Das medizinische Gerät nach Anspruch 2, mit der Eigenschaft, dass der Prozessor (30) ferner konfiguriert ist:

den Atemzustand des Patienten gemäß dem Atemparameter zu erzeugen und anzuzeigen; und/oder
bei Feststellung, dass sich der Atemzustand des Patienten gemäß dem Atemparameter ändert, Hinweisinformationen zu erzeugen

4. Das medizinische Gerät nach Anspruch 1, mit der Eigenschaft, dass der Prozessor (30) ferner konfiguriert ist:

einen empfohlenen Bereich des Beatmungssteuerparameters und einen empfohlenen Bereich des mindestens einen zugehörigen Steuerparameters zu berechnen und den empfohlenen Bereich des Beatmungssteuerparameters und den empfohlenen Bereich des mindestens einen zugehörigen Steuerparameters durch das visuelle Diagramm anzuzeigen; und/oder
das visuelle Diagramm den Beatmungszustand des Patienten anzeigt, indem mindestens einer der aktuellen Werte sowie der Änderungsrichtung des Beatmungsparameters angezeigt werden.

5. Das medizinische Gerät nach Anspruch 1 oder 4, mit der Eigenschaft, dass der Beatmungsparameter einen oder mehrere der Beatmungssteuerparameter oder die zugehörigen Steuerparameter umfasst.

6. Das medizinische Gerät nach Anspruch 5, mit der Eigenschaft, dass das visuelle Diagramm einem Bogendiagramm entspricht, dessen Bogen einen Wertebereich und einen ersten Indikator darstellt, der für einen aktuellen Wert eines entsprechenden Beatmungsparameters steht.

7. Das medizinische Gerät nach Anspruch 6, mit der Eigenschaft,

dass die Zahl am Bogen einen Wert darstellt, der einer Position auf dem Bogen entspricht;
dass der Bogen einen Sicherheitsabschnitt umfasst, der einen empfohlenen Bereich des entsprechenden Beatmungsparameters darstellt, wobei der Sicherheitsabschnitt auf eine Weise dargestellt wird, die sich von anderen Abschnitten des Bogens unterscheidet;
dass das Bogendiagramm ferner einen zweiten Indikator umfasst, der ein Ziel des entsprechenden Beatmungsparameters darstellt, gegebenenfalls eine Zahl, die einen Wert des Ziels des entsprechenden Beatmungsparameters darstellt, neben dem zweiten Indikator angegeben ist;
dass eine Zahl, die den aktuellen Wert des entsprechenden Beatmungsparameters darstellt, neben dem ersten Indikator angegeben ist; wobei das Bogendiagramm ferner einen dritten Indikator umfasst, der eine Änderungsrichtung des entsprechenden Beatmungsparameters darstellt, wobei der dritte Indikator neben der Zahl angeordnet ist, die den aktuellen Wert des entsprechenden Beatmungsparameters darstellt; oder eine zweidimensionale Kurve oder eine Zahlentabelle, die eine Änderungsrichtung der entsprechenden Beatmungsparameter darstellt, ist neben dem Bogendiagramm angeordnet.

8. Das medizinische Gerät nach Anspruch 5, mit der Eigenschaft, dass das visuelle Diagramm einem Histogramm mit einem säulenartigen Segment entspricht, das einen Wertebereich und einen vierten Indikator darstellt, der einem aktuellen Wert eines entsprechenden Beatmungsparameters entspricht.

9. Das medizinische Gerät nach Anspruch 8, mit der Eigenschaft,

dass das säulenartige Segment eine Zahl anzeigt, die einem Wert einer Position auf dem säulenartigen Segment entspricht;
dass das säulenartige Segment einen Sicherheitsabschnitt umfasst, der einen empfohlenen Bereich des entsprechenden Beatmungsparameters darstellt, wobei der Sicherheitsabschnitt auf eine Weise dargestellt wird, die sich von anderen Abschnitten des säulenartigen Segments unterscheidet;
dass das Histogramm ferner einen fünften Indikator umfasst, der ein Ziel des entsprechenden Beatmungsparameters darstellt, gegebenenfalls eine Zahl, die einen Wert des Ziels des entsprechenden Beatmungsparameters darstellt, neben dem fünften Indikator angegeben ist;
dass eine Zahl, die den aktuellen Wert des entsprechenden Beatmungsparameters darstellt, neben dem vierten Indikator angegeben ist; wobei das Histogramm ferner einen sechsten Indikator umfasst, der eine Änderungsrichtung des entsprechenden Beatmungsparameters darstellt, wobei der sechste Indikator neben der Zahl angeordnet ist, die den aktuellen Wert des entsprechenden Beatmungsparameters darstellt; oder eine zweidimensionale Kurve oder eine Zahlentabelle, die eine Änderungsrichtung der entsprechenden Beatmungsparameter darstellt, ist neben dem Histogramm angeordnet.

10. Das medizinische Gerät nach Anspruch 8, mit der Eigenschaft, dass:

der Beatmungsparameter mehrere Beatmungsparameter umfasst, und die Histogramme, die verschiedenen Beatmungsparametern entsprechen, in rotationssymmetrischer Weise oder nebeneinander angeordnet sind; der Prozessor (30) ferner so konfiguriert ist, dass er Werte verschiedener Beatmungsparameter auf entsprechenden Histogrammen zu einem gleichen Zeitpunkt verbindet, um Liniensegmente zu bilden, und diese Liniensegmente zu verschiedenen Zeiten auf unterschiedliche Weise anzeigt; der Prozessor (30) zeigt die Liniensegmente zu verschiedenen Zeiten auf unterschiedliche Weise an, indem er ein Liniensegment zu einem aktuellen Zeitpunkt in einer ersten Farbe anzeigt und Liniensegmente zu vorherigen Zeitpunkten in einer zweiten Farbe mit unterschiedlicher Helligkeit darstellt.

11. Das medizinische Gerät nach Anspruch 5, mit der Eigenschaft, dass das visuelle Diagramm eine zweidimensionale Kurve aufweist; wobei eine horizontale Koordinate der zweidimensionalen Kurve die Zeit darstellt und eine vertikale Koordinate der zweidimensionalen Kurve einem entsprechenden Beatmungsparameter entspricht; wobei die zweidimensionale Kurve eine erste Kurve umfasst, die durch aktuelle Werte des entsprechenden Beatmungsparameters zu unterschiedlichen Zeiten erzeugt wird, und eine zweite Kurve, die durch Ziele der Beatmungsparameter zu unterschiedlichen Zeiten erzeugt wird; wobei die zweite Kurve auf eine Weise dargestellt wird, die sich von der ersten Kurve unterscheidet.

12. Das medizinische Gerät nach Anspruch 11, mit der Eigenschaft, dass die Beatmungsbehandlungsstrategie eine minimale Atemkraftstrategie beinhaltet; wenn festgestellt wird, dass der Patient den Zustand der minimalen Atemkraft erreicht hat, wird ein Bereich, der dies anzeigt, auf der zweidimensionalen Kurve erzeugt.

13. Das medizinische Gerät nach Anspruch 4, mit der Eigenschaft, dass das visuelle Diagramm ein Höhenmesser-Diagramm mit einem vertikalen und skalierten Liniensegment sowie einen Kreis darstellt; wobei der Kreis einen siebten Indikator umfasst, der einen aktuellen Wert eines entsprechenden Beatmungsparameters darstellt, wobei der siebte Indikator eine horizontale Sehne im Kreis ist, und die Skala des Liniensegments, die der Sehne entspricht, den aktuellen Wert des entsprechenden Beatmungsparameters darstellt.

14. Das medizinische Gerät nach Anspruch 13, mit der Eigenschaft, dass:

der Kreis ferner einen empfohlenen Bereich umfasst, der einen empfohlenen Bereich des entsprechenden Beatmungsparameters darstellt, wobei der empfohlene Bereich auf eine Weise dargestellt wird, die sich von anderen Bereichen des Kreises unterscheidet; der Kreis ferner einen achten Indikator umfasst, der ein Ziel des entsprechenden Beatmungsparameters darstellt, wobei der achte Indikator ein horizontaler Durchmesser des Kreises ist, und eine Höhe des Kreises so angeordnet ist, dass die Skala des Liniensegments, die dem horizontalen Durchmesser entspricht, das Ziel des entsprechenden Beatmungsparameters darstellt; und/oder eine zweidimensionale Kurve oder eine Zahlentabelle, die eine Änderungsrichtung des entsprechenden Beatmungsparameters darstellt, neben dem Höhenmesser-Diagramm angeordnet ist.

**Revendications**

1. Dispositif médical, comprenant un appareil d'affichage d'informations de ventilation lequel comprend :

un affichage (50) ; un ou plusieurs capteurs (10), lesquels sont configurés pour acquérir au moins un paramètre respiratoire d'un patient ; et un processeur (30) ; **caractérisé en ce que**, le processeur (30) est configuré pour :

acquérir le au moins un paramètre respiratoire du patient ;

acquérir une cible d'un paramètre de commande de ventilation ;

acquérir une stratégie traitement de ventilation ;

calculer une cible d'au moins un paramètre de commande associé selon la cible du paramètre de commande de ventilation et la stratégie de traitement de ventilation ;

obtenir, selon le au moins un paramètre respiratoire acquis, un paramètre de ventilation qui caractérise un état de ventilation du patient ; et

générer et fournir en sortie un graphique visuel selon le paramètre de ventilation du patient, la cible du paramètre de commande de ventilation et la cible du au moins un paramètre de commande associé, où l'état de ventilation du patient, la cible du paramètre de commande de ventilation et la cible du au moins un paramètre de commande associé sont affichés par le biais du graphique visuel.

2. Dispositif médical selon la revendication 1, **caractérisé en ce que**,

le processeur (30) est en outre configuré pour acquérir la cible du paramètre de commande de ventilation : en déterminant la cible du paramètre de commande de ventilation en réponse à une instruction reçue pour cela, ou en déterminant la cible du paramètre de commande de ventilation selon une ou plusieurs des informations de paramètre respiratoire ou de patient ; et/ou

le processeur (30) est en outre configuré pour acquérir la stratégie de traitement de ventilation en déterminant la stratégie de traitement de ventilation en réponse à une instruction reçue pour cela, ou en déterminant la stratégie de traitement de ventilation selon une ou plusieurs des informations de paramètre respiratoire ou de patient.

3. Dispositif médical selon la revendication 2, **caractérisé en ce que**, le processeur (30) est en outre configuré pour :

générer et afficher un état respiratoire du patient selon le paramètre respiratoire ; et/ou

lors de la détermination qu'un état respiratoire du patient évolue selon le paramètre respiratoire, générer une information d'indication.

4. Dispositif médical selon la revendication 1, **caractérisé en ce que**, le processeur (30) est en outre configuré pour :

calculer une plage recommandée du paramètre de commande de ventilation et une plage recommandée du au moins un paramètre de commande associé ; et afficher la plage recommandée du paramètre de commande de ventilation et la plage recommandée du au moins un paramètre de commande associé par le biais du graphique visuel ; et/ou

le graphique visuel affiche l'état de ventilation du patient en affichant au moins une d'une valeur actuelle et d'une tendance d'évolution du paramètre de ventilation.

5. Dispositif médical selon la revendication 1 ou 4, **caractérisé en ce que**, le paramètre de ventilation comprend un ou plusieurs du paramètre de commande de ventilation ou du paramètre de commande associé.

6. Dispositif médical selon la revendication 5, **caractérisé en ce que**, le graphique visuel comprend un graphique en arc, où le graphique en arc comprend un arc qui représente une plage de valeur et un premier indicateur qui représente une valeur actuelle d'un paramètre de ventilation correspondant.

7. Dispositif médical selon la revendication 6, **caractérisé en ce que**,

l'arc comprend un nombre qui représente une valeur correspondant à une position sur l'arc ;

l'arc comprend un segment de sécurité qui représente une plage recommandée du paramètre de ventilation correspondant, où le segment de sécurité est affiché d'une manière qui est différente du ou des autres segments de l'arc ;

le graphique en arc comprend en outre un deuxième indicateur qui représente une cible du paramètre de ventilation correspondant, optionnellement, un nombre qui représente une valeur de la cible du paramètre de ventilation correspondant, est fourni de manière adjacente au deuxième indicateur ; et/ou

un nombre qui représente la valeur actuelle du paramètre de ventilation correspondant est fourni de manière adjacente au premier indicateur ; où le graphique en arc comprend en outre un troisième indicateur qui représente une tendance d'évolution du paramètre de ventilation correspondant, où le troisième indicateur est adjacent au nombre qui représente la valeur actuelle du paramètre de ventilation correspondant ; ou une courbe bidimensionnelle ou un tableau numérique qui représente une tendance d'évolution des paramètres de ventilation correspondants est agencé de manière adjacente au graphique en arc.

8. Dispositif médical selon la revendication 5, **caractérisé en ce que**, le graphique visuel comprend un histogramme comprenant un segment colonnaire qui représente une plage de valeur et un quatrième indicateur qui représente une valeur actuelle d'un paramètre de ventilation correspondant.

9. Dispositif médical selon la revendication 8, **caractérisé en ce que**,

le segment colonnaire comprend un nombre qui représente une valeur correspondant à une position sur le segment colonnaire ;
le segment colonnaire comprend un segment de sécurité qui représente une plage recommandée du paramètre de ventilation correspondant, où le segment de sécurité est affiché d'une manière qui est différente du ou des autres segments du segment colonnaire ;
l'histogramme comprend en outre un cinquième indicateur qui représente une cible du paramètre de ventilation correspondant, optionnellement un nombre qui représente une valeur de la cible du paramètre de ventilation correspondant est fourni de manière adjacente au cinquième indicateur ; et/ou
un nombre qui représente la valeur actuelle du paramètre de ventilation correspondant, est fourni de manière adjacente au quatrième indicateur; où l'histogramme comprend en outre un sixième indicateur qui représente une tendance d'évolution du paramètre de ventilation correspondant, où le sixième indicateur est adjacent au nombre qui représente la valeur actuelle du paramètre de ventilation correspondant; ou une courbe bidimensionnelle ou un tableau numérique qui représente une tendance d'évolution des paramètres de ventilation correspondants est agencé de manière adjacente à l'histogramme.

10. Dispositif médical selon la revendication 8, **caractérisé en ce que** :
le paramètre de ventilation intègre plusieurs paramètres de ventilation, et les histogrammes qui correspondent aux différents paramètres de ventilation sont répartis de manière symétrique en rotation ou côte à côte ;

où le processeur (30) est en outre configuré pour : relier des valeurs de différents paramètres de ventilation sur des histogrammes correspondants à un même instant pour former des segments, et afficher les segments à différents instants de différentes façons ;
où le processeur (30) affiche les segments à différents instants de différentes façons : en affichant un segment à l'instant présent d'une première couleur, et en affichant des segments à des instants précédents d'une deuxième couleur avec une luminosité différente.

11. Dispositif médical selon la revendication 5, **caractérisé en ce que**, le graphique visuel comprend une courbe bidimensionnelle ; où un axe de coordonnées horizontal de la courbe bidimensionnelle est le temps, et un axe de coordonnées vertical de la courbe bidimensionnelle est un paramètre de ventilation correspondant ;
où la courbe bidimensionnelle comprend un première courbe, qui est générée par des valeurs actuelles du paramètre de ventilation correspondant à différents instants et une deuxième courbe qui est générée par des cibles des paramètres de ventilation à différents instants ; où la deuxième courbe est affichée d'une manière qui est différente de la première courbe.

12. Dispositif médical selon la revendication 11, **caractérisé en ce que**, la stratégie de traitement de ventilation inclut une stratégie de puissance respiratoire minimale ;
lors de la détermination que le patient a atteint l'état de puissance respiratoire minimale, une zone indiquant cela est générée sur la courbe bidimensionnelle.

13. Dispositif médical selon la revendication 4, **caractérisé en ce que**, le graphique visuel comprend un graphique d'altimètre, où le graphique d'altimètre comprend un segment qui est vertical et gradué, et un cercle ; où le cercle comprend un septième indicateur qui représente une valeur actuelle d'un paramètre de ventilation correspondant, où le septième indicateur est une corde horizontale dans le cercle, et la graduation du segment correspondant à la corde représente une valeur actuelle du paramètre de ventilation correspondant.

14. Dispositif médical selon la revendication 13, **caractérisé en ce que**,

le cercle comprend en outre une zone recommandée qui représente une plage recommandée du paramètre de ventilation correspondant ; où la zone recommandée est affichée d'une manière qui est différente de la ou des autres zones du cercle ;

le cercle comprend en outre un huitième indicateur qui représente une cible du paramètre de ventilation correspondant ; où le huitième indicateur est un diamètre horizontal du cercle, et une hauteur du cercle est agencé de sorte que la graduation du segment, qui correspond au diamètre horizontal, est la cible du paramètre de ventilation correspondant ; et/ou

une courbe bidimensionnelle ou un tableau numérique qui représente une tendance d'évolution du paramètre de ventilation correspondant est agencé de manière adjacente au graphique d'altimètre.

FIG.1

FIG.2

Expiratory
branch <u>213a</u>

213c

Patient

211

Inspiratory
branch <u>213b</u>

214a

214b

212

G
A
S

| Memory <u>215</u> | Processor <u>30</u> | Display <u>50</u> |

FIG.3

Expiratory
branch <u>340a</u>

Patient

311

340c

Inspiratory
branch <u>340b</u>

Respiration
assistance
apparatus

320

312

G
A
S

Anesthetic
drug output
apparatus

330

| Memory <u>350</u> | Processor <u>30</u> | Display <u>50</u> |

FIG.4

MV L/min

8.6

6.0

9.0

7.2 ↑

FIG.5 （a）

MV L/min

8.6

6.0

9.0

7.2

MV

t

FIG.5 （b）

MV L/min

8.6

6.0

9.0

7.2

| T | 9:00 | 9:01 | 9:02 | 9:03 |
|---|------|------|------|------|
| MV | 7.6 | 8.0 | 7.7 | 7.2 |

FIG.5 （c）

TV L/min

470

300

480

450 ↑

MV L/min

8.6

6.0

9.0

7.2 ↑

f /min

16

13

20

15 ↑

FIG.6

MV L/min

9.0
8.6

◄ **7.2** ↑

6.0

FIG.7 （a）

MV L/min

9.0
8.6

◄ **7.2** ↑

6.0

MV

t

FIG.7 （b）

MV L/min

9.0
8.6

◄ **7.2** ↑

6.0

| T | 9:00 | 9:01 | 9:02 | 9:03 |
|----|------|------|------|------|
| MV | 7.6 | 8.0 | 7.7 | 7.2 |

FIG.7 （c）

FIG.8

FIG.9

FIG.10

FIG.11

FIG.12

FIG.13

MV $_{\text{L/min}}$

| T | 9:00 | 9:01 | 9:02 | 9:03 |
|------|------|------|------|------|
| MV | 7.6 | 8.0 | 7.7 | 7.2 |

FIG.14

Acquiring at least one respiratory parameter of the patient
who is connected to the medical ventilation device

1000

Acquiring a target of a ventilation control parameter

1010

Acquiring a ventilation treatment strategy

1020

Calculating a target of at least one associated control
parameter according to the target of the ventilation control
parameter and the ventilation treatment strategy

1030

Obtaining, according to the acquired respiratory parameter, a
ventilation parameter which characterizes a ventilation state
of the patient

1040

Generating and outputting a visual graph according to the ventilation parameter of the patient, the
target of the ventilation control parameter and the target of the at least one associated control
parameter, wherein the ventilation state of the patient, the target of the ventilation control parameter
and the target of the at least one associated control parameter are displayed through the visual graph

1050

FIG.15

**REFERENCES CITED IN THE DESCRIPTION**

**Patent documents cited in the description**

- WO 2008039726 A